# EUROPÄISCHE PATENTSCHRIFT

(11) **EP 0 337 467 B1**
(45) Veröffentlichungstag und Bekanntmachung des Hinweises auf die Patenterteilung: **13.07.1994**
(21) Anmeldenummer: 89106644.1
(22) Anmeldetag: 13.04.1989
(51) Int. Cl.: G01N 33/78, G01N 33/96

(54) **Verfahren zur Bestimmung von Thyroxin und dazu geeignete Standardlösung**
Method for the determination of thyroxine and a standard solution therefore
Méthode de détermination de thyroxine et solution standard appropriée

(30) Priorität: 15.04.1988 DE 3812609
(43) Veröffentlichungstag der Anmeldung: 18.10.1989
(73) Patentinhaber: BOEHRINGER MANNHEIM GMBH, 68298 Mannheim (DE)
(72) Erfinder: Kunst, Albert, Dr. Ing., D-6102 Pfungstadt (DE); Rehner, Helmut, Dr., D-8120 Weilheim (DE); Stegmüller, Peter, D-8900 Augsburg 22 (DE)
(74) Vertreter: Huber, Bernhard, Dipl.-Chem.

(56) Entgegenhaltungen:
- US-A- 4 052 504
- US-A- 4 157 895
- CHEMICAL ABSTRACTS, Band 92, Nr. 19, 12 Mai 1980, Columbus, OH (US);I. ILYES et al., Seite 265, Nr. 160009n#
- CLINICAL CHEMISTRY, Band 32, Nr. 5, Mai 1986, Winston-Salem, NC (US); H.R. SCHROEDER et al., Seiten 826-830#
- CLINICAL CHEMISTRY, Band 30, Nr. 10, 1984, Winston-Salem, NC (US); M. ITO et al., Seiten 1682-1685#
- JOURNAL OF NUCLEAR MEDICINE, Band 17, Nr. 4, April 1976, Seiten 321-322, New York, US; P.J.N. HOWORTH et al.

## Beschreibung

Die Erfindung betrifft ein Verfahren zur Bestimmung von Thyroxin (T₄) oder Trijodthyronin (T₃) im Serum unter Verwendung einer Standardlösung sowie diese Standardlösung.

Die Thyreoidhormone liegen im Blut weitgehend in proteingebundener Form vor. Das wichtigste Trägerprotein ist dabei das Thyroxin-bindende Globulin, das als TBG bezeichnet wird, an das ca. 80 % des gesamten im Blut vorliegenden Thyroxins und Trijodthyronins gebunden sind. Etwa 5 bis 10 % des gesamten Thyroxins liegen an Albumin gebunden vor und etwa 10 bis 15 % des gesamten Thyroxins liegen an Präalbumin gebunden vor. Die Gesamtheit dieser Proteine wird auch als Thyroxin-bindendes Protein (TBP) bezeichnet. Nur sehr kleine Anteile an Thyroxin und Trijodthyronin liegen im Blut in freier Form vor, in der Regel sind es ca. 0,03 % des T₄ bzw. 0,3 % des T₃. Nur die freigesetzten Hormone sind physiologisch wirksam, während die gebundenen Hormone T₃ und T₄ als metabolisch inerte Transportform im Blut zirkulieren und als Puffer zur Regelung des Spiegels dienen. Es ist daher wichtig, sowohl den Gesamtthyroxingehalt als auch den Anteil an freiem Thyroxin sowie freiem Trijodthyronin im Blut zu bestimmen, da dieser den funktionellen Thyreoidstatus unabhängig von Änderungen in der Konzentration oder Sättigung des Thyroxin-bindenden Proteins darstellt.

Es sind nun verschiedene Verfahren zur Bestimmung von Gesamtthyroxin (T₄), freiem Thyroxin (FT₄) und Trijodthyronin (T₃) bekannt. Eine Schwierigkeit, die bei allen diesen bekannten Verfahren besteht, ist die Bereitstellung eines zur Eichung geeigneten Standards.

Für die Eichung ist es notwendig, Humanserumstandards mit bestimmten Thyroxin- bzw. Trijodthyroninkonzentrationen und einem definierten FT₄- bzw. FT₃-Gehalt herzustellen. Dazu wurde bisher Humanserum als Ausgangsmaterial verwendet. Dem Humanserum mußte zuerst alles Thyroxin bzw. Trijodthyronin, sowohl in freier als auch in gebundener Form, entzogen werden und dann das so behandelte Humanserum mit definierten Thyroxin- oder Trijodthyroninmengen wieder aufgestockt werden. In der Literatur sind hierzu verschiedene Methoden beschrieben, beispielsweise die Behandlung mit Aktivkohle, mit Ionenaustauschern oder mit trägergebundenen Anti-T₄- bzw. -T₃-Anti-körpern. Diese Verfahren sind alle sehr aufwendig. Außerdem besteht ein Problem darin, daß jedes Humanserum eine andere Zusammensetzung aufweist und daher die einzelnen Chargen große Schwankungen zeigen, so daß die Reproduzierbarkeit zu wünschen übrig läßt.

Ein weiterer Nachteil der bisher bekannten Standardlösungen für die Bestimmung von T₃ und T₄ liegt darin, daß diese Lösungen, die die Zusammensetzung von Humanserum haben, nicht längere Zeit gelagert werden können, da einerseits das enthaltene Protein nach und nach denaturiert wird und andererseits sich das eingestellte Gleichgewicht zwischen gebundenem und freiem T₃ und T₄ verschiebt.

Untersuchungen zum optimalen Proteingehalt von üblichen Standardlösungen sind in Chemical Abstracts, Bd. 92, Nr. 19, Seite 265, Nr. 160009n beschrieben. Die oben geschilderten Probleme werden hierdurch jedoch nicht ausgeräumt.

Ein anderer Ansatz zur Bestimmung der Serumwerte von T₄ wird in Clinical Chemistry, Bd. 32, Nr. 5 (1986), S. 826 - 830 beschrieben. Hierzu ist nach Auffassung der dortigen Autoren eine zusätzliche Bestimmung von TBG nötig, was die Verfahrensführung nicht vereinfacht, sondern - im Gegenteil - weiter kompliziert.

Es war daher Aufgabe der Erfindung, eine Standardlösung für ein Verfahren zur Bestimmung von Thyroxin oder Trijodthyronin zur Verfügung zu stellen, die auch bei längerer Lagerung stabil ist und in einfacher Weise unter Verwendung billiger Ausgangsmaterialien hergestellt werden kann.

Diese Aufgabe wird gelöst durch ein Verfahren zur Bestimmung von Thyroxin oder Trijodthyronin im Serum, das dadurch gekennzeichnet ist, daß man zur Eichung mindestens eine Standardlösung verwendet, die TBG und Thyroxin bzw. Trijodthyronin gelöst in einer Pufferlösung enthält.

Es wurde nun festgestellt, daß sich in einer Lösung, die TBG und Thyroxin oder Trijodthyronin gelöst in einem Puffersystem enthält, das Gleichgewicht zwischen gebundenem und freiem T₄ bzw. T₃ ebenso wie in Humanserum einstellt. Überraschenderweise liefert dabei das erfindungsgemäße unvollständige Gleichgewichtssystem, dem Komponenten der natürlich vorliegenden Thyroxinbindenden Proteine fehlen, sogar bessere Vergleichswerte als eine zuerst Thyreoidhormon-frei gemachte und dann aufgestockte natürliche Matrix auf Basis von Humanserum, so daß also nach entsprechender Eichung derartige Lösungen als Standardlösungen verwendet werden können. Es wird somit erfindungsgemäß für ein Verfahren zur Bestimmung von Thyroxin oder Trijodthyronin eine einfach herzustellende stabile Standardlösung zur Verfügung gestellt.

Zur Bestimmung von Thyroxin und Trijodthyronin sind verschiedene Verfahren bekannt. Bestimmungsverfahren werden beispielsweise beschrieben in Nuc. Compact 16 (1985), 321-327, J. Clin. Immunoassay 7 (1984), 192-205, und J. Clin. Chem. Clin. Biochem. 22 (1984), 895-904. Insbesondere werden zur Bestimmung der Thyreoidhormone Bestimmungsverfahren nach dem Prinzip des Immunoassays verwendet.

Bei einem bekannten Verfahren zur Bestimmung von Thyroxin oder Trijodthyronin im Serum nach dem Prinzip des Immunoassays konkurrieren in der Regel markiertes T₄ bzw. T₃ und T₄ bzw. T₃ der Probe um einen Bindepartner, wie beispielsweise einen Anti-T₄-Antikörper. Durch Einstellen der Reaktionsbedingungen, wie Konzentration einzelner Komponenten, Inkubationsdauer usw., kann entweder das Gesamtthyroxin, -trijodthyronin oder das freie Thyroxin bzw. Trijodthyronin bestimmt werden. Dazu wird die Menge von an den Anti-T₄- bzw. -T₃-Antikörper gebundenem markiertem T₃ oder T₄ bzw. nicht gebundenem markiertem T₃ oder T₄ über die an sich bekannten Nachweisreaktionen bestimmt. Der Gehalt an T₃ oder T₄ kann dann nach der Messung der Markierung errechnet werden, indem man den erhaltenen Wert in Beziehung setzt zu Werten, die man mit bekannten T₃- oder T₄-Konzentrationen erhalten hat. Dazu ist es notwendig, eine Eichkurve zu erstellen, in der das Meßsignal (z.B. der Extinktionswert) gegen die T₃- oder T₄-Konzentration aufgetragen ist. Im Falle des Thyroxins erstellt man zweckmäßigerweise eine Eichkurve aus sechs Thyroxinlösungen mit verschiedenen Konzentrationen, da es keine lineare Beziehung gibt, die eine Zweipunkt-Standardmessung zulassen würde.

Das erfindungsgemäße Verfahren ist sowohl für die Bestimmung von freiem Thyroxin (FT₄) freiem Trijodthyronin (FT₃) als auch von Gesamt-Thyroxin (T₄) und Trijodthyronin (T₃) im Serum geeignet.

Das Thyroxin-bindende Globulin kann aus Humanseren in an sich bekannter Weise gewonnen werden. Bevorzugt enthält die erfindungsgemäß verwendete Standardlösung 5 bis 30 »g/ml TBG. Besonders bevorzugt wird TBG in physiologischer Menge eingesetzt, die im Bereich von 9 bis 20 »g/ml TBG liegt, wie es beispielsweise den Literaturstellen Lab. med. 6 (1982), 27-29, und J. Clin. Chem. Clin. Biochem. 23 (1985) 117-127, zu entnehmen ist.

Bei Verwendung zur Bestimmung von Thyroxin enthält die Standardlösung weiterhin Thyroxin. Das Thyroxin ist bevorzugt in einer Menge von 5 bis 500 ng/ml enthalten. Besonders bevorzugt wird eine physiologische Menge an Thyroxin der Standardlösung zugesetzt, d.h. eine Menge im Bereich von 5 bis 300 ng/ml.

Bei Verwendung zur Bestimmung von Trijodthyronin enthält die Standardlösung zusätzlich noch Trijodthyronin. Das Trijodthyronin wird bevorzugt in einer Menge von 0,5 bis 12 ng/ml eingesetzt. Besonders bevorzugt wird Trijodthyronin in physiologischer Menge, d.h. im Bereich von 0,5 bis 8 ng/ml zugegeben.

Die für das erfindungsgemäße Verfahren verwendete Standardlösung enthält außer TBG und Thyroxin bzw. Trijodthyronin in Pufferlösung vorzugsweise noch Albumin, das die Stabilität der Lösung verbessert. Dabei ist es nicht erforderlich, der erfindungsgemäß bevorzugten Standardlösung Humanserumalbumin zuzusetzen, das natürlich geeignet ist. Ebenso geeignet sind auch die überall leicht erhältlichen und wesentlich günstigeren Albumine, beispielsweise aus Rinder- und Pferdeserum. Bevorzugt wird als Albumin Humanserum-, Rinderserum- oder Pferdeserumalbumin und insbesondere Rinderserumalbumin verwendet. Das Albumin wird bevorzugt in einer Menge von 40 bis 80 mg/ml Lösung verwendet. Besonders bevorzugt wird Albumin in physiologischer Menge, die im Bereich von 40 bis 55 mg/ml liegt, wie aus Documenta Geigy, Wissenschaftl. Tabellen, 7. Ausg. 1975, S. 578, zu entnehmen ist.

Zur Herstellung der Standardlösung wird TBG und gegebenenfalls Albumin in einer Pufferlösung gelöst. Als Puffersystem sind alle Puffer geeignet, die einen pH-Bereich von 6,0 bis 8,0 haben, bevorzugt solche mit einem pH-Wert von 6,5 bis 7,5. Bevorzugt werden GOOD-Puffer eingesetzt, beispielsweise HEPES- oder TRIS-Puffer. Die Pufferkonzentration ist nicht kritisch, bevorzugt wird der Puffer in einer Konzentration von 30 bis 150 mmol/l verwendet. Besonders bevorzugt enthält die Standardlösung 50 bis 100 mmol/l Puffer.

Dieser Lösung wird sodann das Thyroxin oder Trijodthyronin in den geeigneten Konzentrationen zugesetzt. Dabei stellt sich dann ein Gleichgewicht zwischen proteingebundenem und freiem Thyreoidhormon ein analog dem in vivo im Blut herrschenden Gleichgewicht. Diese Lösung ist aufgrund ihrer Zusammensetzung auch bei längerer Lagerung stabil. Da sie aus standardisierten Einzelsubstanzen hergestellt wurde, weist sie eine stets gleichmäßige Zusammensetzung auf und liefert daher reproduzierbare Werte.

Zur Erstellung einer Eichkurve werden mehrere Standardlösungen - in der Regel vier bis sechs - verwendet, deren Thyreoidhormongehalte sich unterscheiden, um aus den erhaltenen Werten eine Kurve zeichnen zu können. In einer bevorzugten Ausführungsform des erfindungsgemäßen Verfahrens wird als erste Standardlösung eine Lösung verwendet, die nur TBG und gegebenenfalls Albumin gelöst in einer Pufferlösung, aber kein Thyreoidhormon enthält. Zur weiteren Kalibrierung werden dann Lösungen mit definierten Hormongehalten eingesetzt.

Ein weiterer Gegenstand der Erfindung ist eine Standardlösung für die Bestimmung von Thyroxin oder Trijodthyronin, die TBG und Thyroxin bzw. Trijodthyronin gelöst in einem Puffersystem enthält.

Bevorzugt enthält die Standardlösung 5 bis 30 »g/ml TBG. Besonders bevorzugt sind in der Standardlösung physiologische Mengen an TBG, d.h. 9 bis 20 »g/ml vorhanden.

In einer bevorzugten Ausführungsform enthält die Standardlösung zur Stabilisierung weiterhin Albumin. Das Albumin ist dabei vorzugsweise in einer Menge von 40 bis 80 mg/ml enthalten. Besonders bevorzugt werden physiologische Mengen an Albumin, d.h. im Bereich von 40 bis 55 mg/ml Albumin, eingesetzt.

Die Standardlösung wird hergestellt, indem man TBG und gegebenenfalls Albumin in einem Puffersystem löst und die jeweils gewünschte Menge an Thyroxin oder Trijodthyronin zugibt. Dabei liegt die Menge an Thyroxin bevorzugt im Bereich von 5 bis 500 ng/ml und besonders bevorzugt im Bereich der physiologischen Menge von 5 bis 300 ng/ml. Wird die Standardlösung zur Bestimmung von Trijodthyronin verwendet, so enthält sie Trijodthyronin bevorzugt in einer Menge von 0,5 bis 12 ng/ml und besonders bevorzugt in physiologischer Menge im Bereich von 0,5 bis 8 ng/ml.

Das Puffersystem weist geeigneterweise einen pH im Bereich von 6,0 bis 8,0 auf und ist bevorzugt ein GOOD-Puffer.

Erfindungsgemäß wird eine Standardlösung mit überlegener Stabilität zur Verfügung gestellt, die in einfacher Weise aus leicht erhältlichen Ausgangsmaterialien hergestellt werden kann.

Die Erfindung wird durch die folgenden Figuren und Beispiele erläutert.
- Figur 1: zeigt eine Eichkurve für verschiedene Thyroxinkonzentrationen, die bei einer Bestimmung von FT₄ in einem heterogenen Enzym-Immunoassay erhalten wurden;
- Figur 2: zeigt eine Eichkurve für verschiedene T₄-Konzentrationen, die bei der Bestimmung von Gesamt-Thyroxin verwendet wurden.
- Figur 3: zeigt eine Eichkurve für verschiedene T₃-Konzentrationen, die bei der Bestimmung von Gesamt-Trijodthyronin verwendet werden können.

### Beispiel 1

Es wurden Lösungen mit verschiedenen Thyroxinkonzentrationen hergestellt. Die Lösungen hatten die folgende Zusammensetzung:
50 mmol/l HEPES-Puffer pH 7,0;
60 mg/ml Rinderserumalbumin;
15 »g/ml Thyroxin-bindendes Globulin (TBG);
zu dieser Lösung wurden steigende Mengen T₄ zugegeben, so daß sich nach Gleichgewichtseinstellung die folgenden Konzentrationen an freiem Thyroxin ergaben: 0; 0,9; 1,5; 2,5; 4,3; 6,5 ng FT₄/dl.

Weiterhin wurden Lösungen mit denselben Zusammensetzungen hergestellt, die jedoch statt Rinderserumalbumin Humanserum- bzw. Pferdeserumalbumin enthielten.

Zur Bestimmung des freien Thyroxins wurden die folgenden Reagenzien verwendet:
- Reagenz 1:: 55 mmol/l Barbitalpuffer pH 7,8;
10 mU/ml T₄-Peroxidase-Konjugat;
- Reagenz 2:: 1,9 mmol/l ABTS (2,2′-Azino-di [3-ethyl-benzthiazolin-sulfonsäure(6)] diammoniumsalz;
3,2 mmol/l Natriumperborat;
100 mmol/l Phosphat/Citratpuffer pH 4,4.

Als Reaktionsgefäße wurden Polystyroltubes verwendet, die mit polyklonalen Anti-T₄-Antikörpern beschichtet waren.

In diese Tubes wurden jeweils 1 ml Reagenz 1 und 20 »l der Standardlösung gegeben und 60 min bei Raumtemperatur inkubiert. Dann wurden die Tubes ausgesaugt und mit Leitungswasser gewaschen. Anschließend wurde 1 ml Reagenz 2 zugegeben und 30 min bei Raumtemperatur inkubiert. Es wurde dann die Extinktion bei 405 nm bzw. 422 nm photometrisch bestimmt.

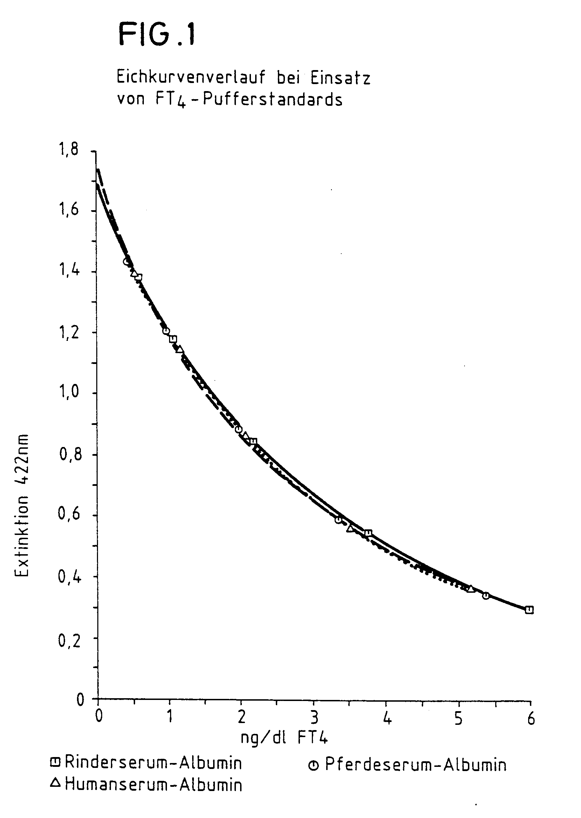

Die den jeweiligen Standardkonzentrationen zugehörigen Extinktionswerte wurden auf Netzpapier aufgetragen und dann eine Kurve gezeichnet, die in Figur 1 gezeigt ist. Aus den Extinktionen der gemessenen Proben können dann aus der Bezugskurve die FT₄-Konzentrationen abgelesen werden. Es zeigt sich, daß Rinderserum- und Pferdeserumalbumin dieselben Werte liefern wie Humanserumalbumin.

### Beispiel 2

Es wurde eine erfindungsgemäße Standardlösung hergestellt und unter den unten angegebenen Bedingungen längere Zeit gelagert. Parallel dazu wurden Standardlösungen nach dem bisher üblichen Verfahren, d.h. Lösen steigender Mengen Thyroxin in T₄-freiem Humanserum, hergestellt und analog gelagert. Es wurde dann überprüft, in wie weit die Standardlösungen dabei stabil bleiben.

Als erfindungsgemäße Standardlösungen wurden die gemäß Beispiel 1 hergestellten Lösungen verwendet, die 0; 0,9; 1,5; 2,5; 4,3 bzw. 6,5 ng FT₄/dl enthielten.

Diese Lösungen wurden anschließend lyophilisiert und drei Wochen bei 35 °C gelagert. Nach der Rekonstitution wurden sie weitere acht Wochen bei 4 °C in Lösung gelagert. Diese Lösungen wurden dann für eine Bestimmung von FT₄, wie sie in Beispiel 1 beschrieben ist, eingesetzt.

Die durchschnittliche Wiederfindung der Standardextinktionen vor und nach der Lagerung ergibt sich aus der folgenden Tabelle I:

**Tabelle I**

| Standard | vor Lagerung | nach Lagerung |
|---|---|---|
| erfindungsgemäße Standardlösung | 100 % | 99 % |
| Humanserum-Standardlösung | 100 % | 89 % |

Wie diese Werte zeigen, ist die erfindungsgemäße Standardlösung sehr viel stabiler als bisher bekannte Humanserumlösungen.

### Beispiel 3

Es wurde die Wiederfindung von FT₄ in 21 Humanseren überprüft. Die Bestimmung des FT₄ wurde bei 20°C bzw. 30°C durchgeführt, wie sie in Beispiel 1 beschrieben ist. Verwendet wurden die gleichen Standardlösungen wie in Beispiel 2. Diese Lösungen wurden lyophilisiert und drei Wochen bei 35 °C gelagert. Anschließend wurden die Lyophilisate rekonstituiert und weitere zwei Wochen bei 25 °C in Lösung gelagert.

Wie die Ergebnisse der Tabelle II zeigen, ist eine temperaturunabhängige Wiederfindung bei Kalibrierung mit der erfindungsgemäßen Standardlösung auch nach längerer Lagerzeit geboten. Mit den instabileren Humanserum-Standardlösungen dagegen werden bei 20°C und 30°C unterschiedliche FT₄-Konzentrationen in Seren gemessen.

**Tabelle II**

| Standard | durchschnittliche Wiederfindung von 21 Humanseren bei 30 °C im Vergleich zur Wiederfindung bei 20 °C |
|---|---|
| erfindungsgemäße Standardlösung | - 0,3 % |
| Humanserum-Standardlösung | - 19,0 % |

### Beispiel 4

Gemäß Beispiel 1 hergestellte Standardlösungen wurden für einen T₄-Test eingesetzt.

Dazu wurden folgende Reagenzien verwendet:
- Reagenz 1:: 120 mmol/l Barbiturat;
18,2 mmol/l Phosphat-Puffer, pH 8,6;
1,27 mmol/l ANS (8-Anilino-1-naphthalinsulfonsäure);
15 mU/ml T₄-Peroxidase-Konjugat;
- Reagenz 2:: Zusammensetzung, wie bei Beispiel 1 beschrieben.

Als Reaktionsgefäße wurden Polystyroltubes verwendet, die mit polyklonalen Anti-T₄-Antikörpern beschichtet waren.

In diese Tubes wurden jeweils 1 ml Reagenz 1 und 20 »l der Standardlösung gegeben und 30 min bei Raumtemperatur inkubiert. Dann wurden die Tubes ausgesaugt und mit Leitungswasser gewaschen. Anschließend wurde 1 ml Reagenz 2 zugegeben und 30 min bei Raumtemperatur inkubiert. Es wurde dann die Extinktion bei 405 nm bzw. 422 nm photometrisch bestimmt.

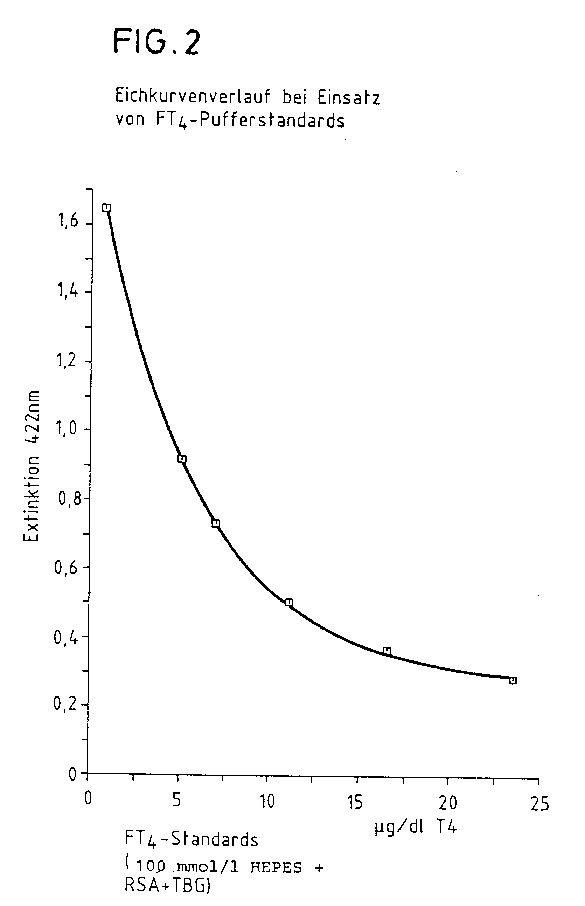

Die den jeweiligen Standardkonzentrationen zugehörigen Extinktionswerte wurden auf Netzpapier aufgetragen und dann eine Kurve gezeichnet, die in Figur 2 gezeigt ist.

### Beispiel 5

Es wurden Lösungen mit verschiedenen Trijodthyronin-Konzentrationen hergestellt. Die Lösungen hatten die folgende Zusammensetzung:
50 mmol/l HEPES-Puffer pH 7,0
60 mg/ml Rinderserumalbumin
15 »g/ml Thyroxin-bindendes Globulin (TBG)
Zu dieser Lösung wurden steigende Mengen T₃ zugegeben. Die so hergestellten Standardlösungen wurden für einen T₃-Test eingesetzt. Dazu wurden die Reagenzien verwendet (Konzentrationen der Gebrauchslösungen).
- Reagenz 1:: 120 mmol/l Barbiturat;
18,2 mmol/l Phosphat-Puffer, pH 8,6;
1,27 mmol/l ANS
(8-Anilino-1-naphthalinsulfonsäure);
12 mU/ml T₃-Peroxidase-Konjugat.
- Reagenz 2:: 100 mmol/l Phosphat-Citrat-Puffer pH 5,0;
1,47 mmol/l Natriumperborat;
9,1 mmol/l ABTS^{®} (2,2′-Azino-di- [3-ethyl-benz-thiazolin-sulfonsäure-(6)]-diammoniumsalz).

Als Reaktionsgefäße wurden Polystyroltubes verwendet, die mit polyklonalen Anti-T₃-Antikörpern beschichtet waren.

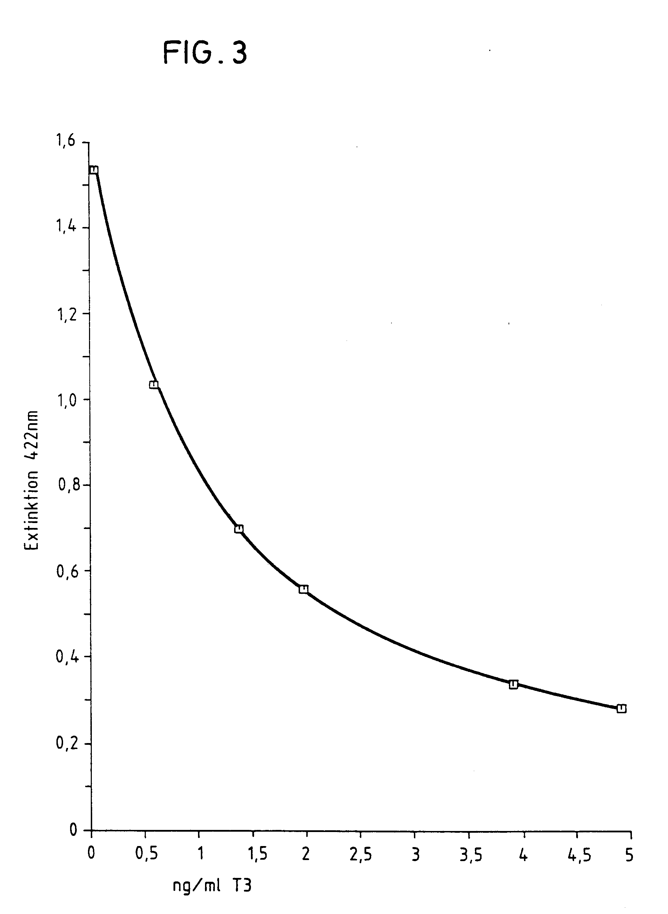

In diese Tubes wurden jeweils 1 ml Reagenz 1 und 100 »l der Standardlösung gegeben und 2 Stunden bei Raumtemperatur inkubiert. Dann wurden die Tubes ausgesaugt und mit Leitungswasser gewaschen. Anschließend wurde 1 ml Reagenz 2 zugegeben und 60 min bei Raumtemperatur inkubiert. Es wurde dann die Extinktion bei 405 nm bzw. 422 nm photometrisch bestimmt. Die Ergebnisse zeigt Fig. 3.

## Patentansprüche

1. Verfahren zur Bestimmung von Thyroxin (T₄) oder Trijodthyronin (T₃) im Serum,
**dadurch gekennzeichnet,**
daß man zur Eichung eine Standardlösung verwendet, die Thyroxin-bindendes Globulin (TBG) und Thyroxin bzw. Trijodthyronin gelöst in einer Pufferlösung enthält.

2. Verfahren nach Anspruch 1,
**dadurch gekennzeichnet,**
daß als weitere Standardlösung eine Lösung verwendet wird, die TBG gelöst in einer Pufferlösung, aber kein Thyroxin oder Trijodthyronin enthält.

3. Verfahren nach Anspruch 1 oder 2,
**dadurch gekennzeichnet,**
daß der Standardlösung zusätzlich Albumin zugesetzt wird.

4. Verfahren nach einem der Ansprüche 1 oder 3,
**dadurch gekennzeichnet,**
daß man eine Standardlösung verwendet, die 5 bis 500 ng/ml und insbesondere 5 bis 300 ng/ml Thyroxin enthält.

5. Verfahren nach einem der Ansprüche 1, 3 oder 4,
**dadurch gekennzeichnet,**
daß man eine Standardlösung verwendet, die 0,5 bis 12 ng/ml und insbesondere 0,5 bis 8 ng/ml Trijodthyronin enthält.

6. Standardlösung für die Bestimmung von Thyroxin oder Trijodthyronin, enthaltend TBG und Thyroxin oder Trijodthyronin gelöst in einer Pufferlösung.

7. Standardlösung nach Anspruch 6,
**dadurch gekennzeichnet,**
daß sie 5 bis 30 »g/ml TBG und 30 bis 150 mmol/l Puffer enthält.

8. Standardlösung nach Anspruch 6 oder 7,
**dadurch gekennzeichnet,**
daß sie zusätzlich Albumin enthält.

9. Standardlösung nach Anspruch 8,
**dadurch gekennzeichnet,**
daß sie zusätzlich 40 bis 80 mg/ml Albumin enthält.

10. Standardlösung nach einem der Ansprüche 7 bis 9,
**dadurch gekennzeichnet,**
daß sie 0,5 bis 12 ng/ml Trijodthyronin und insbesondere 0,5 bis 8 ng/ml Trijodthyronin enthält.

## Claims

1. Method for the determination of thyroxine (T₄) or triiodothyronine (T₃) in serum,
**wherein**
a standard solution is used for the calibration which contains thyroxine-binding globulin (TBG) and thyroxine or triiodothyronine dissolved in a buffer solution.

2. Method as claimed in claim 1,
**wherein**
a solution is used as a further standard solution which contains TBG dissolved in a buffer solution but no thyroxine or triiodothyronine.

3. Method as claimed in claim 1 or 2,
**wherein**
albumin is additionally added to the standard solution.

4. Method as claimed in one of the claims 1 or 3,
**wherein**
a standard solution is used which contains 5 to 500 ng/ml and in particular 5 to 300 ng/ml thyroxine.

5. Method as claimed in one of the claims 1, 3 or 4,
**wherein**
a standard solution is used which contains 0.5 to 12 ng/ml and in particular 0.5 to 8 ng/ml triiodothyronine.

6. Standard solution for the determination of thyroxine or triiodothyronine containing TBG and thyroxine or triiodothyronine dissolved in a buffer solution.

7. Standard solution as claimed in claim 6,
**wherein**
it contains 5 to 30 »g/ml TBG and 30 to 150 mmol/l buffer.

8. Standard solution as claimed in claim 6 or 7,
**wherein**
it in addition contains albumin.

9. Standard solution as claimed in claim 8,
**wherein**
it in addition contains 40 to 80 mg/ml albumin.

10. Standard solution as claimed in one of the claims 7 to 9,
**wherein**
it contains 0.5 to 12 ng/ml triiodothyronine and in particular 0.5 to 8 ng/ml triiodothyronine.

## Revendications

1. Procédé de détermination de la thyroxine (T₄) ou de la triiodothyronine (T₃) dans le sérum, caractérisé en ce que, pour l'étalonnage, on utilise une solution standard qui contient de la globuline liant la thyroxine (GLT) et de la thyroxine ou de la triiodothyronine dissoutes dans une solution tampon.

2. Procédé selon la revendication 1, caractérisé en ce que l'on utilise comme autre solution standard une solution qui contient de la GLT dissoute dans une solution tampon mais qui ne contient ni thyroxine ni triiodothyronine.

3. Procédé selon la revendication 1 ou 2, caractérisé en ce que de l'albumine est ajoutée en plus à la solution standard.

4. Procédé selon l'une des revendications 1 ou 3, caractérisé en ce que l'on utilise une solution standard qui contient 5 à 500 ng/ml et en particulier 5 à 300 ng/ml de thyroxine.

5. Procédé selon l'une des revendications 1, 3 ou 4, caractérisé en ce que l'on utilise une solution standard qui contient 0,5 à 12 ng/ml et en particulier 0,5 à 8 ng/ml de triiodothyronine.

6. Solution standard pour la détermination de la thyroxine ou de la triiodothyronine, contenant de la GLT et de la thyroxine ou de la triiodothyronine dissoutes dans une solution tampon.

7. Solution standard selon la revendication 6, caractérisée en ce qu'elle contient 5 à 30 »g/ml de GLT et 30 à 150 mmol/l de tampon.

8. Solution standard selon la revendication 6 ou 7, caractérisée en ce qu'elle contient en plus de l'albumine.

9. Solution standard selon la revendication 8, caractérisée en ce qu'elle contient en plus 40 à 80 mg/ml d'albumine.

10. Solution standard selon l'une des revendications 7 à 9, caractérisée en ce qu'elle contient 0,5 à 12 ng/ml de triiodothyronine et en particulier 0,5 à 8 ng/ml de triiodothyronine.
